(19) Europäisches Patentamt
European Patent Office
Office européen des brevets

(11) Veröffentlichungsnummer: **0 398 842 A1**

# (12) EUROPÄISCHE PATENTANMELDUNG

(21) Anmeldenummer: **90810344.3**

(51) Int. Cl.$^5$: **C07C 243/38, A01N 37/28**

(22) Anmeldetag: **07.05.90**

(30) Priorität: **16.05.89 CH 1821/89**

(43) Veröffentlichungstag der Anmeldung:
**22.11.90 Patentblatt 90/47**

(84) Benannte Vertragsstaaten:
**BE CH DE FR GB IT LI NL**

(71) Anmelder: **CIBA-GEIGY AG**
**Klybeckstrasse 141**
**CH-4002 Basel(CH)**

(72) Erfinder: **Drabek, Jozef, Dr.**
**Benkenstrasse 12**
**CH-4104 Oberwil(CH)**

(54) **Hydrazid-derivate.**

(57) Neue Triacylhydrazide der Formel I

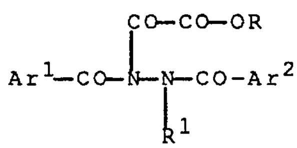

(I)

worin
$Ar^1$ und $Ar^2$ unabhängig voneinander Phenyl oder Naphthyl, oder mit 1 bis 5 der Reste aus der Gruppe Halogen, Nitro, Cyano, 1-6-Alkyl, $C_1$-$C_6$-Haloalkyl, $C_1$-$C_6$-Alkoxy, $C_1$-$C_6$-Haloalkoxy, $C_1$-$C_6$-Alkoxyalkyl, $C_1$-$C_6$-Alkylthio, $C_1$-$C_6$-Alkylsulfinyl, $C_1$-$C_6$-Alkylsulfonyl, Hydroxyl, Carboxyl, $C_1$-$C_6$-Alkylcarbonyl, $C_1$-$C_6$-Alkoxycarbonyl, Methylendioxy, Difluoromethylendioxy, Phenyl, Phenoxy, Pyridyloxy, Phenylthio, Thienyl, Anilido, Benzoyl, Phenylsulfinyl, Phenylsulfonyl, Sulfonamido, $C_2$-$C_6$-Alkenyl, $C_2$-$C_6$-Alkinyl, $C_6$-$C_8$-Cycloalkyl oder Benzyl, unbabhängig voneinander substituiertes Phenyl oder Naphtyl;
R $C_1$-$C_{10}$-Alkyl, durch einen Rest aus der Gruppe Phenyl, $C_3$-$C_8$-Cycloalkyl, Trifluormethyl, Halogen oder 1 bis 2 Cyanreste substituiertes $C_1$-$C_{10}$-Alkyl, $C_2$-$C_{10}$-Alkenyl, $C_3$-$C_{10}$-Alkinyl, $C_3$-$C_8$-Cycloalkyl, Benzyl oder Phenyl und
$R^1$ $C_1$-$C_{10}$-Alkyl, durch einen Rest aus der Gruppe Halogen, Cyano, Nitro, $C_1$-$C_4$-Alkoxy, $C_1$-$C_4$-Alkylcarbonyl, $C_1$-$C_4$-Alkoxycarbonyl, Phenyl oder $C_3$-$C_8$-Cycloalkyl substituiertes $C_1$-$C_{10}$-Alkyl, $C_3$-$C_8$-Cycloalkyl oder Phenyl bedeuten.
Diese Triacylhydrazide haben wertvolle insektizide und akarizide Eigenschaften und können zur Bekämpfung von Schädlingen verwendet werden.

EP 0 398 842 A1

## Hydrazid-Derivate

Die vorliegende Erfindung betrifft neue Triacylhydrazide, ihre Herstellung und diese enthaltende Mittel sowie ihre Verwendung zur Bekämpfung von Schädlingen, vorzugsweise in der Landwirtschaft.

Die erfindungsgemässen Triacylhydrazide entsprechen der Formel I

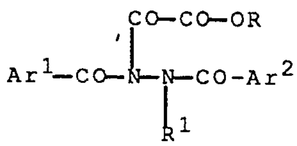

(I)

worin

$Ar^1$ und $Ar^2$ unabhängig voneinander Phenyl oder Naphthyl, oder mit 1 bis 5 der Reste aus der Gruppe Halogen, Nitro, Cyano, 1-6-Alkyl, $C_1$-$C_6$-Haloalkyl, $C_1$-$C_6$-Alkoxy, $C_1$-$C_6$-Haloalkoxy, $C_1$-$C_6$-Alkoxyalkyl, $C_1$-$C_6$-Alkylthio, $C_1$-$C_6$-Alkylsulfinyl, $C_1$-$C_6$-Alkylsulfonyl, Hydroxyl, Carboxyl, $C_1$-$C_6$-Alkylcarbonyl, $C_1$-$C_6$-Alkoxycarbonyl, Methylendioxy, Difluoromethylendioxy, Phenyl, Phenoxy, Pyridyloxy, Phenylthio, Thienyl, Anilido, Benzoyl, Phenylsulfinyl, Phenylsulfonyl, Sulfonamido, $C_2$-$C_6$-Alkenyl, $C_2$-$C_6$-Alkinyl, $C_3$-$C_8$-Cycloalkyl oder Benzyl, unabhängig voneinander substituiertes Phenyl oder Napthyl;

R $C_1$-$C_{10}$-Alkyl, durch einen Rest aus der Gruppe Phenyl, $C_3$-$C_8$-Cycloalkyl, Trifluormethyl, Halogen oder 1 bis 2 Cyanoreste substituiertes $C_1$-$C_{10}$-Alkyl, $C_1$-$C_{10}$-Alkenyl, $C_3$-$C_{10}$-Alkinyl, $C_2$-$C_8$-Cycloalkyl, Benzyl oder Phenyl; und

$R^1$ $C_1$-$C_{10}$-Alkyl, durch einen Rest aus der Gruppe Halogen, Cyano, Nitro, $C_1$-$C_4$-Alkoxy, $C_1$-$C_4$-Alkylcarbonyl, $C_1$-$C_4$-Alkoxycarbonyl, Phenyl oder $C_3$-$C_8$-Cycloalkyl substituiertes $C_1$-$C_{10}$-Alkyl, $C_3$-$C_8$-Cycloalkyl oder Phenyl bedeuten.

Gute Wirkung wurde mit denjenigen Verbindungen der Formel I erreicht, in denen $Ar^1$ und $Ar^2$ unabhängig voneinander Phenyl- oder Naphthyl, oder mit 1 bis 5 der Reste aus der Gruppe Halogen, Nitro, Cyano, $C_1$-$C_6$-Alkyl, $C_1$-$C_6$-Haloalkyl, $C_1$-$C_6$-Alkoxy, $C_1$-$C_6$-Haloalkoxy, Hydroxyl, $C_1$-$C_6$-Alkoxycarbonyl, Methylendioxy oder Phenoxy unabhängig voneinander substituiertes Phenyl oder Naphtyl;

R $C_1$-$C_{10}$-Alkyl unsubstituiert oder durch Phenyl-, $C_3$-$C_8$-Alkyl- oder Cyano substituiert oder $C_3$-$C_8$-Cycloalkyl und

$R^1$ $C_1$-$C_{10}$-Alkyl, unsubstituiert oder durch Halogen oder Phenyl substituiert, $C_3$-$C_8$-Cyclohexyl oder Phenyl bedeuten.

Bevorzugt sind erfindungsgemäss diejenigen Verbindungen der Formel I, in denen $Ar^1$ und $Ar^2$ Phenyl oder durch Halogen, Nitro, Cyano, $C_1$-$C_6$-Alkyl, $C_1$-$C_6$-Haloalkyl, $C_1$-$C_6$-Alkoxy, $C_1$-$C_5$-Haloalkoxy, Hydroxyl, $C_1$-$C_6$-Alkoxycarbonyl oder Phenyl substituiertes Phenyl;

R $C_1$-$C_5$-Alkyl unsubstituiert oder durch Halogen oder Cyano substituiert, Phenyl, Benzyl oder $C_3$-$C_6$-Cycloalkyl bedeuten.

Wegen ihrer biologischen Wirkung hervorzuheben sind vor allem die Verbindungen der Formel I, in denen $Ar^1$ und $Ar^2$ unabhängig voneinander Phenyl oder Tolyl; R $C_1$-$C_4$-Alkyl; und $R^1$ tert.-Butyl bedeuten; wobei $Ar^1$ und $Ar^2$ vorzugsweise Phenyl bedeuten, speziell die folgenden Verbindungen:

N,N'-Dibenzoyl-N-tert.butyl-N'-tert.butoxyloxalyl-hydrazid,
N,N'-Dibenzoyl-N-tert.butyl-N'-ethoxyoxalyl-hydrazid,
N,N'-Dibenzoyl-N-tert.butyl-N'-isopropoxyoxalyl-hydrazid,
N,N'-Dibenzoyl-N-tert.butyl-N'-methoxyoxalyl-hydrazid,
N,N'-Dibenzoyl-N-tert.butyl-N'-n-propoxyoxalyl-hydrazid,
N,N'-Dibenzoyl-N-tert.butyl-N'-n-butoxyoxalyl-hydrazid,
N-(4-methylbenzoyl)-N'-benzoyl-N-tert.butyl-N'-methoxyoxalyl-hydrazid und
N-Benzoyl-N'-(4-methylbenzoyl)-N-tert.butyl-N'-methoxyoxalyl-hydrazid.

Unter dem Begriff Alkyl allein oder im Zusammenhang wie z.B. Alkoxy, Alkylsulfonyl etc. sind im Rahmen der vorliegenden Erfindung geradkettige und verzweigte Alkylreste und je nach Zahl der angegebenen Kohlenstoffatome beispielsweise folgende Gruppen zu verstehen: Methyl, Aethyl, Propyl, Butyl, Pentyl usw. sowie ihre Isomeren, wie z.B. Isopropyl, Isobutyl, tert.-Butyl, Isoamyl, tert. Pentyl usw. Unter Halogen sind Fluor, Chlor, Brom und Jod, vorzugsweise Fluor und Chlor, zu verstehen.

Unter dem Begriff Haloalkyl im Rahmen der vorliegenden Erfindung sind geradkettige und verzweigte

Alkylreste, wie Methyl, Aethyl, n-Propyl, Isopropyl und die isomeren Butylreste zu verstehen, die mit bis zu 9 unterschiedlichen oder gleichartigen Halogenatomen substituiert sind, wobei es sich um perhalogenierte Alkylreste oder auch um solche handeln kann, deren Wasserstoffatome nur teilweise durch Halogen substituiert sind. Als Halogenalkyle sind beispielsweise die Perfluoralkylradikale Trifluormethyl, Pentafluoräthyl oder Heptafluor-n-propyl zu nennen.

Die erfindungsgemässen Verbindungen der Formel I sind neu; sie können nach an sich bekannten Verfahren hergestellt werden; wie z.B.:

Die Triazylhydrazide der Formel I sind erhältlich gemäss einem ersten Syntheseweg, indem man ein Oxalylhydrazid der Formel II

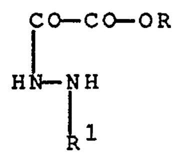

(II)

worin R und $R^1$ die unter der Formel I vorstehend angegebenen Bedeutungen haben, vorzugsweise in einem inerten organischen Lösungsmittel, in Gegenwart der mindestens äquimolaren Menge einer Base, zunächst mit einem reaktionsfähigen Benzoyl- oder Naphthoylester der Formel III

$Ar^1$-CO-X (III),

worin $Ar^1$ die unter der Formel I angegebene Bedeutung hat und X Halogen, Phenylsulfonyl, $C_1$-$C_4$-Alkylsulfonyl oder den Anhydridrest -O-CO-$Ar^1$ bedeutet, umsetzt, und das erhaltene Hydrazid der Formel IV

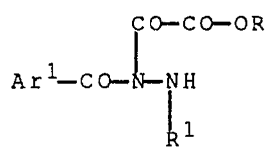

(IV),

worin $Ar^1$, R und $R^1$ die unter der Formel I angegebenen Bedeutungen haben, dann vorzugsweise in einem inerten organischen Lösungsmittel, in Gegenwart der mindestens äquimolaren Menge einer Base, mit einem reaktionsfähigen Benzoyl- oder Naphthoylester der Formel IIIa

$Ar^2$-CO-X (IIIa),

worin $Ar^2$ die unter der Formel I gegebenen Bedeutung hat und X Halogen Phenylsulfonyl, $C_1$-$C_4$-Alkylsulfonyl oder den Anhydridrest-O-CO-$Ar^2$ bedeutet, umsetzt.

Wenn im Endprodukt der Formel I $Ar^1$ und $Ar^2$ identisch sind, kann das Hydrazid der Formel II mit mindestens der doppelten Menge eines reaktionsfähigen Benzoyl- oder Naphthoylesters oder eines Anhydrides der Formel III resp. IIIa umgesetzt werden.

Als inerte organische Lösungsmittel für diese Umsetzungen kommen Aether, Tetrahydrofuran, Diisopropyläther, Dioxan, Ketone, wie Aceton und Methyläthylketon, Acetonitril, Toluol oder chlorierte Kohlenwasserstoffe wie Chloroform, Methylenchlorid, Tetrachlorkohlenstoff, Aethylenchlorid in Frage.

Als Basen kommen sowohl anorganische, wie Natrium- und Kaliumhydroxyd, Natriumhydrid, Alkali- und Erdalkalimetallcarbonate, sowie Bicarbonate, als auch organische Basen, tertiäre Amine wie Trimethylamin, Träthylamin, N-Methylpyridin oder auch Pyridin usw. in Frage.

Die Umsetzungen werden bei einer Temperatur zwischen -20° bis 100° C, vorzugsweise zwischen 0 und 50° C durchgeführt.

Die als Ausgangsprodukte benötigten Hydrazine der Formel II sind bekannt oder können nach bekannten Methoden hergestellt werden, siehe z.B. I.S. Zmuszkovicez und M.E. Greig, J. Med. Chem. 4, 259 (1961).

Ein anderer Weg zur Herstellung der Triacylhydrazide der Formel I besteht darin, dass man ein Hydrazid der Formel V

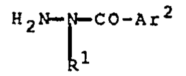 (V),

worin $Ar^2$ und $R^1$ die unter der Formel I angegebenen Bedeutungen haben, vorzugsweise in einem inerten organischen Lösungsmittel, in Gegenwart der mindestens äquimolaren Menge einer Base, mit einem reaktionsfähigen Oxalsäureester der Formel VI

Y-CO-CO-OR (VI),

worin R die unter der Formel I angegebene Bedeutung hat und Y Halogen, vorzugsweise Chlor oder Brom, $C_1$-$C_4$-Alkoxy oder Benzyloxy bedeutet, umsetzt und das so erhaltene Oxalyl-Hydrazid der Formel VII

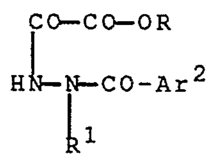 (VII),

worin $Ar^2$, R und $R^1$ die unter der Formel I angegebenen Bedeutungen haben, vorzugsweise in einem inerten organischen Lösungsmittel und in Gegenwart der mindestens äquimolaren Menge einer Base, mit einem reaktionsfähigen Benzoyl- oder Naphthoylester oder -anhydrid der Formel III

$Ar^1$-CO-X (III)

worin $Ar^1$ die unter der Formel I angegebene Bedeutung hat und X Halogen Phenylsulfonyl, $C_1$-$C_4$-Alkylsulfonyl oder den Anhydridrest O-CO-$Ar^2$ bedeutet, umsetzt. Als inerte Lösungsmittel kommen für diese Umsetzungen Toluol, die Xylole, Aether wie Diäthyl-oder Diisopropyläther, Tetrahydrofuran oder Dioxan, Ketone wie Aceton, Methyläthylketon in Betracht. Als Basen kommen sowohl anorganische wie Natrium- oder Kaliumhydroxid, Natriumhydrid, Alkyli- oder Erdalkalimetallcarbonate und -bicarbonate, als auch organische Basen, tertiäre Amine wie Trimethylamin, Triäthylamin, Methylpyridin oder auch Pyridin usw. in Frage.

Die Umsetzungen finden bei einer Temperatur zwischen -20 bis +100° C, vorzugsweise zwischen 0° bis 50° C statt.

Die als Ausgangsprodukt benötigten Hydrazide der Formel V sind entweder bekannt oder können nach bekannten Methoden hergestellt werden [vgl. P.AS. Smith, J.M. Clegg and J. Lakritz, J. Org. Chem. 33 - (1958) p.1595].

Schliesslich können die Triacylhydrazine der Formel I auch hergestellt werden, indem man ein Diacylhydrazid der Formel

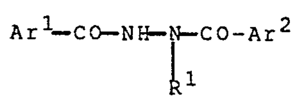 (VIII)

worin $Ar^1$, $Ar^2$ und $R^1$ die unter der Formel I gegebenen Bedeutungen haben, vorzugsweise in einem inerten organischen Lösungsmittel und in Gegenwart der mindestens äquimolaren Menge einer Base, mit einem reaktionsfähigen Oxalsäureester der Formel VI

Y-CO-CO-OR (VI),

worin R die unter der Formel I angegebene Bedeutung hat und Y Halogen, vorzugsweise Chlor oder Brom, $C_1$-$C_4$-Alkoxy oder Benzyloxy bedeutet, umsetzt. Für diese Umsetzung kommen als Lösungsmittel Aether wie Diäthyl- oder Diisopropyläther, Tetrahydrofuran oder Dioxan, aber auch Toluol oder die Xylole in Betracht. Als Basen kommen sowohl anorganische, wie Natrium- oder Kaliumhydroxid, Natriumhydrid, Alkali- und Erdalkalimetallcarbonate sowie -bicarbonate, als auch organische Basen, tertiäre Amine wie Trimethylamin, Triäthylamin, N-Methylpyridin oder Pyridin in Frage. Die Reaktionstemperatur für diese Umsetzung liegt zwischen -20° und +100° C, vorzugsweise zwischen 0° und 60° C.

Die als Ausgangsprodukte benötigten Diacylhydrazide der Formel VIII sind entweder bekannt oder können nach bekannten Methoden hergestellt werden, siehe dazu Helv. Chim. Acta. 61 (1978) p.1977. EP-A 228 564 oder EP-A 236,618.

Insektizid wirksame Hydrazide sind in den offengelegten Europäischen Patentanmeldungen Nr. 228,564, 236,618 und 245,950 beschrieben. Es handelt sich bei diesen bekannten Verbindungen um Diacylhydrazid-Derivate. Die erfindungsgemässen Verbindungen unterschieden sich als Triacylhydrazide strukturell von diesen bekanten Stoffen. Es wurde nun überraschenderweise gefunden, dass die erfindungsgemässen Verbindungen der Formeln I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Schädligen.

Die erfindungsgemässen Verbindungen der Formeln I weisen eine besonders ausgeprägte Wirkung gegen pflanzenschädigende Akariden (Spinnmilben: z.B. der Familien Tetranychidae, Tarsonemidae, Eriophyidae, Tyroglyphidae und Glycyphagidae) und auch gegen ektoparasitäre Akarinen (Milben und Zecken: z.B. der Familien Ixodidae, Argasidae, Sarcoptidae und Dermanyssidae), welche Nutztiere befallen, auf. Einige der erfindungsgemässen Verbindungen zeichnen sich durch gute akarizid-ovizide Atkivität und durch Blattpenetrationswirkung aus. Die erfindungsgemässen Verbindungen eignen sich vor allem zur Bekämpfung der folgenden, Obst- und Gemüsekulturen befallenden Milbenspezies: Tetranychus urticae, Tetranychus cinnabarinus, Panonychus ulmi, Bryobia rubrioculus, Panonychus citri, Eriophyes pyri, Eriophyes ribis, Eriophyes vitis, Tarsonemus pallidus, Phyllocoptes vitis und Phyllocoptruta oleivora.

Darüberhinaus wurde festgestellt, dass die Verbindungen der Formel I auch insektizide Eigenschaften besitzen und demzufolge zur Bekämpfung von pflanzenschädigenden Insekten, z.B. der Ordnungen Lepidoptera, Coleoptera, Heteroptera, Diptera, Orthoptera und Homoptera, besonders geeignet sind.

Neben ihrer sehr günstigen Wirkung gegenüber Fliegen, wie z.B. Musca domestica, und Mückenlarven eignen sich Verbindungen der Formel I zur Bekämpfung von pflanzenschädigenden Frassinsekten, in Zier- und Nutzpflanzungen, insbesondere in Baumwollkulturen (z.B. Spodoptera, littoralis und Heliothis virescens) sowie in Gemüsekulturen (z.B. Leptinotarsa decemlineata und Plutella xylostella), im Reis (z.B. Chilo suppresalis).

Die gute pesitizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Deri vate, Formamidine, Harnstoffe, Carbomate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, d.h. die die Wirkstoffe der Formel I bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden erfindungsgemässen

Wirkstoffes oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss-oder Tallöl gewonnen werden können. Ferner sind als Tenside auch die Fettsäure-methyltaurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atom. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenyl-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome in Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten überlicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tenside handelt es sich vor allem um quaternäre Ammoniumsalze, welche als N-Substituenten mindestens einen Alkylrest mit 8 bis 22 C-Atomen enthalten und als weitere Substituenten niedrige, gegenebenfalls halogenierte Alkyl-, Benzyl- oder niedrige Hydroxyalkylreste aufweisen. Die Salze liegen vorzugsweise als Halogenide, Methylsulfate oder Aethylsulfate vor z.B. das Stearyltrimethylammoniumchlorid oder das Benzyl-di-(2-chloräthyl)-äthylammoniumbromid.

Die in der Formulierungstecknik gebräuchlichen Tenside sind u.a. in folgenden Publikationen beschrieben:

"McCutcheon's Detergents and Emulsifiers Annual" MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides (oberflächenaktiven Mittels), wobei sich die %-Angaben auf das Gewicht beziehen. Während als Handelsware eher konzentrierte Mittel bevorzugt werden, verwendet der Endverbraucher in der Regel verdünnte Mittel bzw. Zubereitungen, die wesentlich geringere Wirkstoffkonzentration, wie z.B. 0,1 bis 1000 ppm, aufweisen. Gewöhnlich beträgt die eingesetzte Aufwandmenge der erfindungsgemässen Wirkstoffe der Formel I - insbesondere für landwirtschaftliche Kulturflächen - 0,025 bis 1,0 kg/ha, vorzugsweise 0,1 bis 0,5 kg/ha, beispielsweise 0,1 bis 0,25 kg/ha.

Die Mittel können auch weitere Zusätze wie Stabilisatoren, Entschäumer, Viskositätregulatoren, Bindemittel, Haftmittel sowie Dünger oder andere Wirkstoffe zur Erzielung spezieller Effekte enthalten.

In den folgenden experimentellen Beispielen sind die (unkorrigierten) Schmelzpunkte in °C angegeben.

<u>Beispiel 1: Herstellung von N,N′-Dibenzoyl-N-tert.butoxyoxalyl-N′-tert.-butyl-hydrazid</u>

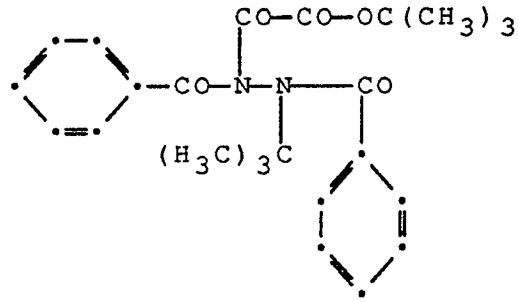

Zu einer Lösung von 6,7 g N,N′-Dibenzoyl-N-tert.butyl-hdyrazid in 200 ml Tetrahydrofuran gibt man unter Rühren bei einer Temperatur von 0 bis 5 °C portionenweise 1,4 g Natriumhydrid (55 %-ige Mineralödispersion). Nachdem die Wasserstoffentwicklung aufgehört hat, tropft man bei einer Temperatur von 0° bis 10 °C, 4.8 g tert.Butoxyoxalychlorid zur Reaktonslösung. Nachdem alles zugegeben ist, wird das Reaktionsgemisch eine Stunde bei Raumtemperatur weitergerührt, dann filtriert und das Filtrat eingeengt. Das zurückbleibende Oel wird in Methylenchlorid gelöst, zweimal mit Wasser gewaschen, getrocknet und eingeengt. Das erhaltene Rohprodukt wird zur Reinigung über eine mit Kieselgel (Merck 60) gefüllte Säule von 15 cm Höhe und 8 cm Durchmesser, mit Hexan/Methylenchlorid 1:1 als Eluat chromatographiert.

Nach Einengen des Eluates wird der Rückstand in Hexan verrieben. Man erhält so das Titelprodukt als farblose Kristalle, welche bei 115-116 °C schmelzen (Verbindung Nr. 1.001).

Beispiel 2: Herstellung von N,N′-Dibenzoyl-N′-ethoxyoxalyl-N′-tert.butylhydrazid

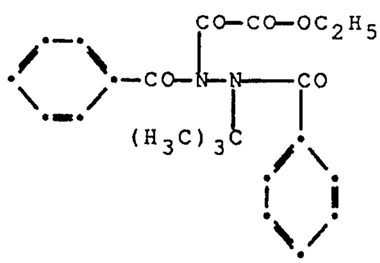

Zu einer Suspension von 12,46 g tert. Butylhydrazin-Hydrochlorid in 200 ml absolutem Aether tropft man unter Rühren bei 5 bis 10 °C, 22,3 g Triäthylamin und rührt weitere 30 Minuten bei 5° bis 10 °C. Anschliessend tropft man 13,65 g Oxylsäureethylesterchlorid bei 5° bis 10 °C in die Suspension und rührt weitere 2 Stunden bei 10 °C. Das Reaktionsgemisch wird dann abgenutscht, der Aether vom Filtrat abdestiliert und der Rückstand im Hochvakuum destilliert. Man erhält so N-Ethoxyoxalyl-N′-tert.butylhydrazid, welches einen Siedepunkt von 135 °C/0.05 mbar hat.

Zu einer Lösung von 9,8 g N-Ethoxyoxalyl-N′-tert.butylhydrazid in 50 ml Toluol tropft man bei 0° bis 5 °C unter Rühren gleichzeitig 15,5 g Benzoesäurechlorid und 2,5 ml einer 20%igen wässrigen Natriumhydroxidlösung. Nachdem alles zugetropft ist, rührt man noch 2 Stunden bei Raumtemperatur weiter, verdünnt dann mit Wasser, extrahiert die organische Phase, wäscht sie mit Wasser trocknet sie und destilliert das Toluol ab. Der Rückstand wird zur Reinigung über eine mit Kieselgel Merck 60 gefüllte Kolonne chromatographiert. Als Eluat wird Hexan/Aether/Isopropanol 3:5:1 genommen. Nach verdampfen des Lösungsmittels verbleibt das Titelprodukt als gelbliches viskoses Oel mit einem Brechungsindex $n_D^{40}$ 1.5362 (Verbindung Nr. 1.002).

In analoger Weise wie vorstehend angegeben werden auch die in der Tabelle 1 aufgeführten Verbindungen hergestellt.

Tabelle 1

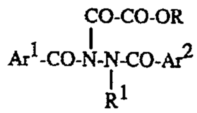

| Nr. | $Ar^1$ | $Ar^2$ | $R^1$ | R | phys. Daten |
|---|---|---|---|---|---|
| 1.001 | Phenyl | Phenyl | $C(CH_3)_3$ | $C(CH_3)_3$ | Smp. 115-116°C |
| 1.002 | Phenyl | Phenyl | $C(CH_3)_3$ | $C_2H_5$ | $n_D^{40}$ 1,5362 |
| 1.003 | Phenyl | Phenyl | $C(CH_3)_3$ | $CH_3$ | Smp. 87-92°C |
| 1.004 | Phenyl | Phenyl | $C(CH_3)_3$ | $CH(CH_3)_2$ | Smp. 66-68°C |
| 1.005 | Phenyl | Phenyl | $C(CH_3)_3$ | $CH_2CH_2CH_3$ | $n_D^{40}$ 1,5292 |
| 1.006 | Phenyl | Phenyl | $C(CH_3)_3$ | $C_4H_9$-n | Smp. 92-96°C |
| 1.007 | Phenyl | Phenyl | $C(CH_3)_3$ | Phenyl | |
| 1.008 | Phenyl | Phenyl | $C(CH_3)_3$ | Cyclohexyl | |
| 1.009 | 4-Chlor-phenyl | Phenyl | $C(CH_3)_3$ | $C_2H_5$ | |
| 1.010 | p-Tolyl | Phenyl | $C(CH_3)_3$ | $C_2H_5$ | |
| 1.011 | 4-Methoxy-phenyl | Phenyl | $C(CH_3)_3$ | $C_2H_5$ | |
| 1.012 | Phenyl | 4-Chlor-phenyl | $C(CH_3)_3$ | $C_2H_5$ | |
| 1.013 | Phenyl | p-Tolyl | $C(CH_3)_3$ | $C_2H_5$ | |
| 1.014 | p-Tolyl | p-Tolyl | $C(CH_3)_3$ | $C_2H_5$ | |
| 1.015 | 4-Nitro-phenyl | Phenyl | $C(CH_3)_3$ | $CH_3$ | |
| 1.016 | Phenyl | 4-Cyano-phenyl | $C(CH_3)_3$ | $CH_3$ | |
| 1.017 | Phenyl | 4-(1,1,2,-3,3,3-Hexa-fluoroprop-yl)phenyl | $C(CH_3)_3$ | $C_2H_5$ | |

Tabelle 1 (Fortsetzung)

| Nr. | $Ar^1$ | $Ar^2$ | $R^1$ | R | phys. Daten |
|---|---|---|---|---|---|
| 1.018 | 4-Trifluor-methyl-phenyl | Phenyl | $C(CH_3)_3$ | $CH_3$ | |
| 1.019 | 3,4-Di-chloro-phenyl | Phenyl | $C(CH_3)_3$ | $C(CH_3)_3$ | |
| 1.020 | 4-Phenoxy-phenyl | Phenyl | $C(CH_3)_3$ | $C_2H_5$ | |
| 1.021 | α-Naphthyl | Phenyl | $C(CH_3)_3$ | $C_2H_5$ | |
| 1.022 | 2,6-Di-fluoro-phenyl | Phenyl | $C(CH_3)_3$ | $C(CH_3)_3$ | |
| 1.023 | Phenyl | 2,4,6-Tri-fluoro-phenyl | $C(CH_3)_3$ | $C(CH_3)_3$ | |
| 1.024 | Phenyl | Phenyl | Cyclohexyl | $C_2H_5$ | |
| 1.025 | Phenyl | β-Naphthyl | $C(CH_3)_3$ | $C_2H_5$ | |
| 1.026 | 2-Chlor-4-nitro-phenyl | Phenyl | $C(CH_3)_3$ | $C(CH_3)_3$ | |
| 1.027 | p-Tolyl | p-Tolyl | $C(CH_3)_3$ | $C(CH_3)_3$ | |
| 1.028 | 3,4-Meth-ylendioxy-phenyl | Phenyl | $C(CH_3)_3$ | $C(CH_3)_3$ | |
| 1.029 | 2-Ethoxy-carbonyl-phenyl | Phenyl | $C(CH_3)_3$ | $C_2H_5$ | |
| 1.030 | 4-Methyl-sulfonyl-phenyl | Phenyl | $C(CH_3)_3$ | $C_2H_5$ | |

Tabelle 1 (Fortsetzung)

| Nr. | $Ar^1$ | $Ar^2$ | $R^1$ | R | phys. Daten |
|---|---|---|---|---|---|
| 1.031 | Phenyl | 4(1,1,2,2-Tetrafluoroethoxy-phenyl | $C(CH_3)_3$ | $C(CH_3)_3$ | |
| 1.032 | 4-Trifluormethyl-phenyl | 4-Trifluoromethyl-phenyl | $CH(CH_3)_3$ | $CH_3$ | |
| 1.033 | 3,4-Dichloro-phenyl | Phenyl | $CH(CH_3)_2$ | $CH_3$ | |
| 1.034 | 2,4-Difluoro-phenyl | Phenyl | $C(CH_3)_3$ | $C_2H_5$ | |
| 1.035 | o-Tolyl | Phenyl | $C(CH_3)_3$ | $CH_3$ | |
| 1.036 | Phenyl | Phenyl | $C(CH_3)_2C_2H_5$ | $C_2H_5$ | |
| 1.037 | Phenyl | Phenyl | $C(CH_3)_2$Phenyl | $CH_3$ | |
| 1.038 | Phenyl | Phenyl | $C(CH_3)_3$ | Benzyl | |
| 1.039 | Phenyl | Phenyl | $C(CH_3)_3$ | $CH_2CF_3$ | |
| 1.040 | 4-Cyano-phenyl | Phenyl | $C(CH_3)_3$ | $C(CH_3)_3$ | |
| 1.041 | 2,4-Dichloro-phenyl | 2,4-Dichloro-phenyl | $C(CH_3)_3$ | $CH_3$ | |
| 1.042 | Phenyl | Phenyl | $CH(CN)CH_2CN$ | $CH_3$ | |
| 1.043 | Phenyl | Phenyl | $CH_2CH(CH_3)CN$ | $C(CH_3)_3$ | |
| 1.044 | 4-Dimethyl-amino-phenyl | Phenyl | $C(CH_3)_3$ | $CH_3$ | |
| 1.045 | p-Tolyl | Phenyl | $C(CH_3)_3$ | $CH_3$ | Smp. 114-116°C |
| 1.046 | Phenyl | p-Tolyl | $C(CH_3)_3$ | $CH_3$ | Smp. 91-93°C |

Beispiel 3: Wirkung gegen Tetranychus uriticae (OP-sensible) und Tetranychus cinnabarinus (OP-tolerant)

Die Primärblätter von Phaseolus vulgaris-Pflanzen wurden 12 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt (Mischpopulation). Die Toleranz bezieht sich auf die Verträglichkeit gegenüber Diazinon.

Die so behandelten infestierten Pflanzen werden mit einer Versuchslösung in Emulsionsform enthaltend 400 ppm der zu prüfenden Verbindung bis zur Tropfnässe besprüht.

Nach 24 Stunden und wiederum nach 6 (T.urticae) bzw. 7 Tagen (T.cinnabarinus) werden Imagines und Larven (alle beweglichen Stadien) sowie abgelegte Eier unter dem Binokular auf lebende und tote Individuen ausgewertet.

Man verwendet pro Testpezies eine Pflanze. Während des Versuchsverlaufs stehen die Pflanzen in Gewächshauskabinen bei 25 °C mit etwa 50-60 % rel. Luftfeuchtigkeit.

Die geprüften Verbindungen der Tabelle 1 zeigen in diesem Versuch gute Wirkung gegen Tetranychus urticae und Tetranychus cinnabarinus.

Beispiel 4: Ovizide Wirkung auf Tetranychus urticae (OP-resistent)

Eingetopfte Phaseolus vulgaris-Pflanzen im Primärblatt-Stadium werden jeweils zweimal mit 30 Weibchen von Teranychus urticae besiedelt. Nach 24-stündiger Eiablage werden die Weibchen mit einer Saugpumpe (Wasserstrahlpumpe) von den Pflanzen entfernt, so dass auf diesen nur noch die abgelegten Milbeneier verbleiben.

Die mit den Milbeneiern infestierten Pflanzen werden dann mit einer wässrigen Emulsion enthaltend 400 ppm der zu prüfenden Verbindung bis zur Tropfnässe besprüht und 5 Tage bei 25 °C und etwa 50 % relativer Luftfeuchtigkeit gehalten. Nach dieser Zeit wird die prozentuale Mortalität der Eier und geschlüpften Larven durch Auszählen bestimmt.

Die geprüften Verbindungen der Tabelle 1 zeigen gute Wirkung in obigem Test.

Beispiel 5: Pflanzenmitizide Blattpenetration-Wirkung auf Tetranychus cinnabarinus

Für den Versuch werden eingetopfte Buschbohnenpflanzen im Primärblatt-Stadium, die mit Tetranychus cinnabarinus infestiert sind, verwendet. Die Besiedlung mit den Milben erfolgt 1 Tag vor der Wirkstoffapplikation.

Die Blattoberseiten der mit diesen Milben infestierten Versuchspflanzen werden mit einer Emulsionszubereitung enthaltend 400 ppm des zu prüfenden Wirkstoffes besprüht. Nach dem Antrocknen des Sprühbelages wird jeweils der Rand der Blattoberseite einer Anzahl befallener Blätter mit einem Wulst aus zähflussigem Leim (Raupenleim) abgegrenzt, um ein Ueberlaufen der Milben von der Blattunterseite auf die Blattoberseite zu verhindern.

Die behandelten Pflanzen werden dann im Gewächshaus bei einer Temperatur von 25 °C bis 27 °C und einer relativen Luftfeuchtigkeit von ca. 50 % gehalten. Sechs Tage nach der Wirkstoffapplikation wird festgestellt, ob eine translaminare Wirkung, d.h. Blattpenetration des Wirkstoffs von der Blattoberseite zur Blattunterseite, eingetreten war, und zwar durch Bestimmung der prozentualen Mortalität der Eier und larvalen sowie adulten Stadien.

Die geprüften Verbindungen der Tabelle 1 zeigen gute Wirkung in obigem Test.

Beispiel 6: Wirkung gegen Panonychus ulmi (OP und Carb. resistent)

Eingetopfte Apfelsämlinge mit etwa 20-30 Blättern werden mit jeweils 60 adulten Weibchen von Panonychus ulmi besiedelt. Nach sieben Tagen werden die infestierten Pflanzen mit einer wässrigen Emulsion enthaltend 400 ppm der zu prüfenden Verbindung bis zur Tropfnässe besprüht. Die behandelten Pflanzen werden dann während weiterer 14 Tage bei 25 °C und etwa 50 % relativer Luftfeuchtigkeit im Gewächshaus aufgestellt.

Nach dieser Zeit erfolgt die Auswertung, indem man 20 Blätter pro Pflanze entnimmt, die Milbenpopulation mittels einer Abbürst-Vorrichtung von den entnommenen Blättern entfernt und unter dem Binocular nach Eiern, postembryonalen Stadien und Adulten auszählt. Bewertet wird die prozentuale Reduktion der Milbenpopulation gegenüber unbehaldelten Kontrollen.

Die geprüften Verbindungen der Tabelle 1 zeigen gute Wirkung in obigem Test.

Beispiel 7: Wirkung gegen tierparasitäre Milben

Von mit Dermanyssus gallinae befallenen Hühnern werden Ansätze bestehend aus etwa 50 Milben verschiedener Stadien (Mischpopulation: Larven, Nymphen und Adulte) ent nommen. Die Milbenansätze

werden jeweils mit einer wässrigen Emulsion, Suspension bzw. Lösung enthaltend 800 ppm des zu prüfenden Wirkstoffes benetzt. Hierzu werden die Milbenansätze in einem Reagenzglas mit der den Wirkstoff enthaltenden flüssigen Zubereitung übergossen; die Flüssigkeit wird anschliessend mit Hilfe eines Wattebausches aufgesogen. Die so benetzten und behandelten Milben verbleiben während 72 Stunden in dem Reagenzglas. Nach dieser Zeit wird die Abtötung der behandelten Milben gegenüber unbehandelten Kontrollansätzen ermittelt.

Die geprüften Verbindungen der Tabelle 1 zeigen gute Wirkung im obligen Test.

Beispiel 8: Wirkung gegen Zecken: Abtötungswirkung in verschiedenen Entwicklungsstadien

Als Testobjekte pro Ansatz werden nicht vollgesogene Larven (jeweils ca. 50), Nymphen (jeweils 5) oder Adulte (jeweils 5) der Zeckenarten Rhipicephalus bursa, Amblyomma hebraeum und Boophilus microplus verwendet. Die Testtiere (in der angegebenen Anzahl) werden für kurze Zeit in 2 bis 3 ml einer wässriger Emulsion der zu untersuchenden Substanzen mit einer Konzentration von 400 ppm getaucht, die sich in einem Reagenzglas befindet. Die Reagenzgläser werden dann mit einem Wattepfropfen verschlossen und 10 Minuten nach dem Eintauchen der Testtiere geschüttelt. Dabei wird die Wirkstoff-Emulsion von den Wattepfropfen aufgesogen und die benetzten Testtiere in den so kontaminierten Reganzgläsern belassen. Die Auswertung (% - Mortalität) erfolgt für Laven nach 3 Tagen und für Nymphen und Adulte nach 14 Tagen.

Die geprüften Verbindungen der Tabelle 1 zeigen gute Wirkung im obigen Test.

Beispiel 9: Wirkung gegen Zecken: Eiablagehemmung

Als Testtiere werden vollgesogene adulte Weibchen der Rinderzecke Boophilus microplus verwendet. Es werden je 10 Zecken eines OP-resistenten Stammes (z.B. vom Biarra-Stamm) und eines normal empfindlichen Stammes (z.B. vom Yeerongpilly-Stamm) behandelt. Die Zecken werden auf mit Doppelklebeband bezogenen Platten fixiert und dann entweder mit wässrigen Emulsionen bzw. mit Lösungen enthaltend 800 ppm der zu untersuchenden Verbindung oder eines ihrer Salze benetzt oder mit einem mit diesen Flüssigkeiten getränkten Wattebausch in Berührung gebracht. Anschliessend werden sie in einem klimatisierten Raum bei konstanten Bedingungen aufbewahrt. Die Auswertung erfolgt nach drei Wochen. Es wird prozentuale Hemmung der Ablage von fertilen Eiern gegenüber unbehandelten Kontrollen ermittelt.

Die geprüften Verbindungen der Tabelle 1 zeigen gute Wirkung im obligen Test.

Beispiel 10: Wirkung gegen Musca domestica

Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Die geprüften Verbindungen der Tabelle 1 zeigen gute Wirkung im obigen Test.

Beispiel 11: Insektizide Frassgift-Wirkung

Baumwoll-, Soja- und Chinakohlpflanzen (ca. 20 cm hoch) werden mit wässrigen Wirkstoffemulsionen (erhalten aus einem 25%igen emulgierbaren Konzentrat) besprüht, wobei die Wirkstoffemulsionen 200, 100, 50 and 12.5 ppm der jeweils zu prüfenden Verbindung enthalten.

Nach dem Antrocknen des Belages werden die Pflanzen mit je 10 Larven von Spodoptera littoralis im

dritten larvalen Stadium, Heliothis virescens im ersten und dritten larvalen Stadium, und Crocidolonia binotalis im zweiten larvalen Stadium besetzt. Der Versuch wird bei 24° C und 60 % relativer Luftfeuchtigkeit durchgeführt. In Abständen von jeweils 48 und 96 Stunden werden Mortalität sowie Entwicklungs- und Häutungsstörungen der angesetzten Larven bestimmt.

Die Verbindung 1.002 zeigte im obigen Test folgende Wirkung:

Nach Antrocknen des Belages abgetrennte und mit Larven von Heliothis virescens im ersten larvalen Stadium besetzte Sojablätter in Petrischalen zeigten 6 Tage nach Besiedlung eine Mortalität von 80-100 % bei der Wirkstoffemulsion von 12.5 ppm.

Nach Antrocknen des Belages auf Sojapflanzen angesetzte Larven von Heliothis virescens im dritten larvalen Stadium zeigten nach 5 Tagen eine Mortalität von 80-100 % bei der Wirkstoffkonzentration von 100 ppm.

Nach Antrocknen des Belages auf Sojapflanzen angesetzte Larven von Spodoptera littoralis im dritten larvalen Stadium zeigten nach 5 Tagen eine Mortalität von 80-100 % bei der Wirkstoffkonzentration von 200 ppm.

Nach Antrocknen des Belages auf Chinakohlpflanzen angesetzte Larven von Crocidolomia binotalis im zweiten larvalen Stadium zeigten nach 5 Tagen eine Mortalität von 80-100 % bei der Wirkstoffkonzentration von 50 ppm.

Beispiel 12: Ovizide Wirkung auf Spodoptera littoralis

Auf Filterpapier abgelegte Eier von Spodoptera littoralis werden aus dem Papier ausgeschnitten und in eine 0,05 Gew.%ige Lösung des Wirkstoffes in einem Aceton-Wasser-Gemisch (1:1) getaucht. Die so behandelten Eiablagen werden dann aus diesem Gemisch herausgenommen und bei 28° C und 60 % relativer Feuchtigkeit in Kunststoffschalen deponiert.

Nach 5 Tagen wird die Schlupfrate, d.h. die Anzahl Larven, die sich aus den behaldelten Eiern entwickelt haben, bestimmt.

Die geprüften Verbindungen der Tabelle I zeigen gute Wirkung im obigen Test.

Beispiel 13: Ovizide Wirkung auf Heliothis virescens und Leptinotarsa decemlineata

Entsprechende Mengenanteile einer benetzbaren pulverförmigen Formulierung, enthaltend 25 Gew.% des zu prüfenden Wirkstoffes, werden mit jeweils so viel Wasser vermischt, dass sich eine wässrige Emulsion mit einer Wirkstoffkonzentration von 800 ppm ergibt.

In die wirkstoffhaltigen Emulsionen werden eintägige Eigelege von Heliothis auf Cellophan® bzw. Eigelege von Leptinotarsa auf Kartoffelblättern während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgelegt.

Die geprüften Verbindungen der Tabelle I zeigen gute Wirkung in obigem Test.

Beispiel 14: Wirkung gegen Lucilia sericata

Zu 9 ml eines Zuchtmediums wird bei 50° C 1 ml einer 0,5% Aktivsubstanz enthaltenden wässrigen Zubereitung gegeben. Nun werden ca. 30 frisch geschlüpfte Lucilia sericataLarven zum Zuchtmedium hinzugefügt. Nach 48 and 96 Stunden wird die insektizide Wirkung durch Ermittlung der Abtötungsrate festgestellt.

Die geprüften Verbindungen der Tabelle 1 zeigen gute Wirkung im obligen Test.

Beispiel 15: Wirkung gegen Aëdes aegypti

Auf die Oberfläche von 150 ml Wasser, das sich in einem Behälter befindet, wird so viel einer 0,1 %igen acetonischen Lösung des Wirkstoffes pipettiert, dass eine Konzentration von 400 ppm erhalten wird. Nach Verdunsten des Acetons wird der Behälter mit 30 bis 40 2-tägigen Ae"des-Larven beschickt. Nach 2 und 7 Tagen wird die Mortalität geprüft.

Die geprüften Verbindungen der Tabelle 1 zeigen gute Wirkung im obigen Test.

Beispiel 16: Formulierungen für Wirkstoffe der Formel I resp. Kombinationen dieser Wirkstoffe mit andern Insektiziden oder Akariziden (% = Gewichtsprozent):

| 1. Spritzpulver | a) | b) | c) |
|---|---|---|---|
| Wirkstoff gemäss Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

| 2. Emulsions-Konzentrat | |
|---|---|
| Wirkstoff gemäss Tabelle 1 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff gemäss Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| 4. Extruder-Granulat | |
|---|---|
| Wirkstoff gemäss Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

| 5. Umhüllungs-Granulat | |
|---|---|
| Wirkstoff gemäss Tabelle 1 | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthyleglykol angefeuchtete Kaolin gleichmässig aufgetragen. Auf diese Weise erhält man staubfreie Umhüllungs-Granulate.

| 6. Suspensions-Konzentrat | |
|---|---|
| Wirkstoff gemäss Tabelle 1 | 40 % |
| Aethylenglykol | 10 % |
| Nonylphenolpolyäthylenglykoläther (5 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Ansprüche

1. Triacylhydrazine der Formel I

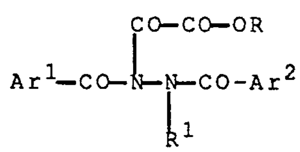

(I)

worin

$Ar^1$ und $Ar^2$ unabhängig voneinander Phenyl oder Naphthyl, oder mit 1 bis 5 der Reste aus der Gruppe Halogen, Nitro, Cyano, 1-6-Alkyl, $C_1$-$C_6$-Haloalkyl, $C_1$-$C_6$-Alkoxy, $C_1$-$C_6$-Haloalkoxy, $C_1$-$C_6$-Alkoxyalkyl, $C_1$-$C_6$-Alkylthio, $C_1$-$C_6$-Alkylsulfinyl, $C_1$-$C_6$-Alkylsulfonyl, Hydroxyl, Carboxyl, $C_1$-$C_6$-Alkylcarbonyl, $C_1$-$C_6$-Alkoxycarbonyl, Methylendioxy, Difluoromethylendioxy, Phenyl, Phenoxy, Pyridyloxy, Phenylthio, Thienyl, Anilido, Benzoyl, Phenylsulfinyl, Phenylsulfonyl, Sulfonamido, $C_2$-$C_6$-Alkenyl, $C_2$-$C_6$-Alkinyl, $C_3$-$C_8$-Cycloalkyl oder Benzyl, unbabhängig voneinander substituiertes Phenyl oder Naphtyl;

R $C_1$-$C_{10}$-Alkyl, durch einen Rest aus der Gruppe Phenyl, $C_3$-$C_8$-Cycloalkyl, Trifluormethyl, Halogen oder 1 bis 2 Cyanreste substituiertes $C_1$-$C_{10}$-Alkyl, $C_2$-$C_{10}$-Alkenyl, $C_3$-$C_{10}$-Alkinyl, $C_3$-$C_8$-Cycloalkyl, Benzyl oder Phenyl und

$R^1$ $C_1$-$C_{10}$-Alkyl, durch einen Rest aus der Gruppe Halogen, Cyano, Nitro, $C_1$-$C_4$-Alkoxy, $C_1$-$C_4$-Alkylcarbonyl, $C_1$-$C_4$-Alkoxycarbonyl, Phenyl oder $C_3$-$C_8$-Cycloalkyl substitueirtes $C_1$-$C_{10}$-Alkyl, $C_3$-$C_8$-Cycloalkyl oder Phenyl bedeuten.

2. Triacylhydrazide gemäss Anspruch 1 der Formel I, worin

$Ar^1$ und $Ar^2$ unabhängig voneinander Phenyl- oder Naphthyl, oder mit 1 bis 5 der Reste aus der Gruppe Halogen, Nitro, Cyano, $C_1$-$C_6$-Alkyl, $C_1$-$C_6$-Haloalkyl, $C_1$-$C_6$-Alkoxy, $C_1$-$C_6$-Haloalkoxy, Hydroxyl, $C_1$-$C_6$-Alkoxycarbonyl, Methylendioxy oder Phenoxy unabhängig voneinander substituiertes Phenyl oder Naphtyl;

R $C_1$-$C_{10}$-Alkyl unsubstituiert oder durch Phenyl-, $C_3$-$C_8$-Alkyl- oder Cyano substituiert oder $C_3$-$C_8$-Cycloalkyl und
$R^1$ $C_1$-$C_{10}$-Alkyl, unsubstituiert oder durch Halogen oder Phenyl substituiert, $C_3$-$C_8$-Cyclohexyl oder Phenyl bedeuten.

3. Triacylhydrazide gemäss Anspruch 1 der Formel I, worin,
$Ar^1$ und $Ar^2$ Phenyl oder durch Halogen, Nitro, Cyano, $C_1$-$C_6$-Alkyl, $C_1$-$C_6$-Haloalkyl, $C_1$-$C_6$-Alkoxy, $C_1$-$C_6$-Haloalkoxy, Hydroxyl, $C_1$-$C_6$-Alkoxycarbonyl oder Phenyl substituiertes Phenyl;
R $C_1$-$C_5$-Alkyl unsubstituiert oder durch Halogen oder Cyano substituiert, Phenyl-$C_1$-$C_5$-alkyl oder $C_3$-$C_8$-Cycloalkyl und
$R^1$ $C_1$-$C_5$-Alkyl, unsubstituiert oder durch Halogen oder Cyano substituiert, Phenyl, Benzyl oder $C_3$-$C_8$-Cycloaklyl bedeuten.

4. Triacylhydrazide gemäss Anspruch 1 der Formel I, worin
$Ar^1$ und $Ar^2$ unabhängig voneinander Phenyl oder Tolyl;
R $C_1$-$C_4$-Alkyl und
$R^1$ tert.Butyl bedeuten.

5. Triacylhydrazide gemäss Anspruch 4 der Formel I, worin $Ar^1$ und $Ar^2$ Phenyl bedeuten.

6. Triacylhydrazide gemäss Anspruch 1 aus der Gruppe
N,N'-Dibenzoyl-N-tert.butyl-N'-tert.butoxyloxalyl-hydrazid,
N,N'-Dibenzoyl-N-tert.butyl-N'-ethoxyoxalyl-hydrazid,
N,N'-Dibenzoyl-N-tert.butyl-N'-isopropoxyoxalyl-hydrazid,
N,N'-Dibenzoyl-N-tert.butyl-N'-methoxyoxalyl-hydrazid,
N,N'-Dibenzoyl-N-tert.butyl-N'-propxyoxalyl-hydrazid,
N,N'-Dibenzoyl-N-tert.butyl-N'-butoxyoxalyl-hydrazid,
N-(4-Methylbenozyl)-N'-benzoyl-N-tert.butyl-N'-methoxyoxalyl-hydrazid und
N-Benozyl-N'-(4-methylbenzoyl)-N-tert.butyl-N'-methoxyoxalyl-hydrazid.

7. Verfahren zur Herstellung der Triacylhydrazide der Formel I gemäss Anspruch 1, dadurch gekennzeichnet, dass man ein Oxalylhydrazid der Formel II

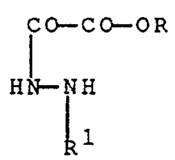

(II)

worin R und $R^1$ die im Anspruch 1 angegebenen Bedeutungen haben, zunächst mit einem reaktionsfähigen Benzoyl-oder Naphthoylester der Formel III
$Ar^1$-CO-X (III),
worin $Ar^1$ die im Anspruch 1 gegebene Bedeutung hat und X Halogen, Phenylsulfonyl, $C_1$-$C_4$-Alkylsulfonyl oder den Anhydridrest-O-CO-$Ar^1$ bedeutet, umsetzt, und das so erhaltene Hydrazid der Formel IV

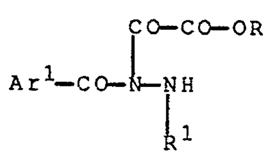

(IV),

worin $Ar^1$, R und $R^1$ die im Anspruch 1 angegebenen Bedeutungen haben, dann mit einem reaktionsfähigen Benzoyl- oder Naphthoylester der Formel IIIa
$Ar^2$-CO-X (IIIa)
worin $Ar^2$ die im Anspruch 1 angegebene Bedeutung hat und X Halogen, Phenylsulfonyl, $C_1$-$C_4$-Alkylsulfonyl oder den Anhydridrest-O-CO-$Ar^2$ bedeutet, umsetzt.

8. Verfahren zur Herstellung von Triacylhydraziden der Formel I gemäss Anspruch 1, worin $Ar^1$ und $Ar^2$ die gleiche Bedeutung haben, dadurch gekennzeichnet, das man ein Oxalylhydrazid der Formel II

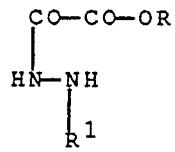

(II)

worin R und $R^1$ die im Anspruch 1 angegebenen Bedeutungen haben,mit mindestens der zweifachen stöchiometrischen Menge eines reaktionsfähigen Benzoyl- oder Naphthoylester der Formel III umsetzt.
$Ar^1$-CO-X (III),
worin $Ar^1$ die im Anspruch 1 angegebene Bedeutung hat und X Halogen, Phenylsulfonyl, $C_1$-$C_4$-Alkylsulfonyl oder den Anhydridrest-O-OC-$Ar^1$ bedeutet.

9. Verfahren zur Herstellung der Triacylhydrazide der Formel I gemäss Anspruch 1, dadurch gekennzeichnet, dass man ein Hydrazid der Formel V

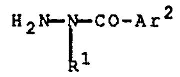

(V),

worin $Ar^2$ und $R^1$ die im Anspruch 1 angegebenen Bedeutungen haben, mit einem reaktionsfähigen Oxalsäureester der Formel VI
Y-CO-CO-OR (VI),
worin R die im Anspruch 1 angegebene Bedeutung hat und Y Halogen, vorzugsweise Chlor oder Brom, $C_1$-$C_4$-Alkoxy oder benzyloxy bedeutet, umsetzt und das so erhaltene Oxalyl-Hydrazid der Formel VII

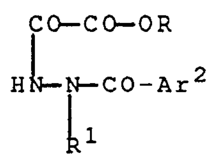

(VII),

worin $Ar^2$, R und $R^1$ die im Anspruch 1 angegebenen Bedeutungen haben, sodann mit einem reaktionsfähigen Benzoyl- oder Nahphthoylester oder -anhydrid der Formel III
$AR^1$-CO-X (III),
worin $Ar^1$ die im Anspruch 1 angegebene Bedeutung hat und X Halogen Phenylsulfonyl, $C_1$-$C_4$-Alkylsulfonyl oder den Anhydridrest O-CO-$Ar^2$ bedeutet, umsetzt.

10. Verfahren zur Herstellung der Triacylhydrazide der Formel I gemäss Anspruch 1, dadurch gekennzeichnet, dass man ein Diacylhydrazid der Formel VIII

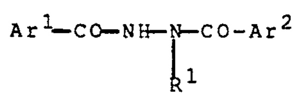

(VIII)

worin $Ar^1$, $Ar^2$ und $R^1$ die im Anspruch 1 angegebenen Bedeutungen haben, mit einem reaktionsfähigen Oxalsäureester der Formel VI
Y-CO-CO-OR (VI)
worin R die im Anspruch 1 angegebene Bedeutung hat und Y Halogen, $C_1$-$C_4$-Alkoxy oder Benzyloxy bedeutet, umsetzt.

11. Mittel zur Bekämpfung von Schädlingen, welches neben inerten Zusätzen und Formulierungshilfsmitteln als aktive Komponente eine pestizid wirksame Menge eines Triacylhydrazides der Formel I gemäss Anspruch 1 enthält.

12. Verwendung einer Verbindung gemäss Anspruch 1 zur Bekämpfung von Insekten und Vertretern der Ordnung Akarina.

13. Verwendung gemäss Anspruch 12 zur Bekämpfung von pflanzenschädigenden Insekten.

14. Verwendung gemäss Anspruch 13 zur Bekämpfung von pflanzenschädigenden Insekten Baumwoll- und Gemüsekulturen.

15. Verwendung gemäss Anspruch 14 zur Bekämpfung von pflanzenschädigenden Lepidopteren.

16. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung gemäss Anspruch 1 oder mit einem Mittel enthaltend neben Zusatz- und Trägerstoffen eine pestizid wirksame Menge dieser Verbindung, in Kontakt bringt oder behandelt.

17. Verfahren gemäss Anspruch 16 zur Bekämpfung von pflanzenschädigenden Insekten.

18. Verfahren gemäss Anspruch 17 zur Bekämpfung von pflanzenschädigenden Insekten in Baumwoll- und Gemüsekulturen.

19. Verfahren gemäss Anspruch 18 zur Bekämpfung von pflanzenschädigenden Lepidopteren.

Europäisches Patentamt

# EUROPÄISCHER RECHERCHENBERICHT

Numm... der Anmeldung

EP 90 81 0344

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 286 746 (ROHM et al.)<br>* Anspruch 1 *<br>--- | 1 | C 07 C 243/38<br>A 01 N 37/28 |
| D,A | EP-A-0 245 950 (ROHM et al.)<br>* Anspruch 1 *<br>--- | 1 | |
| A | PATENT ABSTRACTS OF JAPAN<br>vol. 3, no. 156 (E-161), 21 December 1979; & JP-A-54136821 (KONISHIROKU SHASHIN KOGYO) 24.10.1979<br>--- | 1 | |
| A | CHEMICAL ABSTRACTS<br>Band 110, Nr. 3, 16 Januar 1989, Seite 543, Zusammenfassung Nr. 23787r, Columbus, Ohio, US; H. WEBER et al.: "Ring opening of 3(2H)-pyrazolones by osication with periodate" & Arch. Pharm. (Weinheim, Ger.), 1988, Band 321, Nr. 9, Seiten 551-553<br>--- | 1 | |
| | | | RECHERCHIERTE SACHGEBIETE (Int. Cl.5) |
| A | CHEMICAL ABSTRACTS<br>Band 100, 7.-21. Mai 1984, Seite 530, Zusammenfassung Nr. 156547c, Columbus, Ohio, US; O.P. SHAVAIKA et al.: "Acetyl group migration in 1,3,4-oxidiazolium salts" & Khim. Geterotskl. Soedin., 1983, Nr. 11, Seite 1563<br>----- | 1 | C 07 C 243/38<br>A 01 N 37/28 |

Der vorliegende Recherchenbericht wurde für alle Patentansprüche erstellt

| Recherchenort | Abschlußdatum der Recherche | Prüfer |
|---|---|---|
| BERLIN | 20-08-1990 | KAPTEYN H G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)